# EUROPÄISCHE PATENTSCHRIFT

(11) **EP 4 203 902 B1**
(45) Veröffentlichungstag und Bekanntmachung des Hinweises auf die Patenterteilung: **11.03.2026**
(21) Anmeldenummer: 21755907.9
(22) Anmeldetag: 27.07.2021
(51) Int. Cl.: A61K 8/27, A61K 8/37, A61K 8/49, A61Q 5/00

(54) **HAARBEHANDLUNGSMITTEL MIT ANTISCHUPPENWIRKUNG**
HAIR CARE COMPOSITION WITH A ANTIDANDRUFF EFFECT
COMPOSITION DE SOIN CAPILLAIRE AVEC UN EFFET ANTI-PELLICULAIRE

(30) Priorität: 27.08.2020 DE 102020210831
(43) Veröffentlichungstag der Anmeldung: 05.07.2023
(73) Patentinhaber: Henkel AG & Co. KGaA, 40589 Düsseldorf (DE)
(72) Erfinder: BATTERMANN, Marlene, 21271 Asendorf (DE); KERL, Sylvia, 25474 Bönningstedt (DE)
(86) Internationale Anmeldenummer: PCT/EP2021/071000
(87) Internationale Veröffentlichungsnummer: WO 2022/042979

(56) Entgegenhaltungen:
- WO-A1-2020/160741
- US-A1- 2014 120 048
- US-A1- 2019 184 209
- "Antimicrobial activity of fatty acid esters and combinations thereof ED - Darl Kuhn", IP.COM, IP.COM INC., WEST HENRIETTA, NY, US, 23 January 2020 (2020-01-23), XP013185531, ISSN: 1533-0001

## Beschreibung

Die vorliegende Erfindung betrifft Haarbehandlungsmittel umfassend eine spezielle Kombination von Antischuppenwirkstoffen, die Verwendung der Mittel zur Reinigung von Haaren und zur Prophylaxe, Verminderung, Beseitigung und Linderung von Schuppen auf behaarten Körperoberflächen.

Reinigungsmittel für die Haut und die Haare, wie sie beispielsweise als flüssige Seifen, Shampoos, Duschbäder, Schaumbäder, Dusch- und Waschgele im Handel erhältlich sind, müssen ein gutes Reinigungsvermögen aufweisen, hautverträglich sein und selbst bei häufiger Anwendung nicht zu starker Entfettung oder Trockenheit von Haut und Haaren führen.

Ein wichtiger Aspekt bei der Haarreinigung und -pflege ist zudem die Bekämpfung von Schuppen, denn das Auftreten von Schuppen auf der Kopfhaut oder anderen behaarten Körperregionen wird als Zeichen mangelnder Pflege angesehen. Darüber hinaus geht mit der Schuppenbildung meist ein störender Juckreiz einher, der Kratzreaktionen hervorrufen und zu Verletzungen der betroffenen Hautpartien führen kann, welche wiederum die Basis für Infektionen und pathogene Erreger bilden können.

Die Anforderungen an kosmetische Mittel zur Schuppenbekämpfung sind hoch, denn sie sollen nicht nur Reinigung, Pflege und Antischuppenwirksamkeit gewährleisten, sondern auch schnell - optimalerweise bereits ab der ersten Anwendung - und nachhaltig wirken.

Aus der Literatur ist eine Vielzahl haarkosmetischer Mittel bekannt, die die Schuppenbildung verhindern oder reduzieren. Zur Auswahl stehen üblicherweise Mittel in der Form von Shampoos, Lotionen oder Haarwässern. Die internationale Patentanmeldung WO-A-2020/160741 offenbart

Haarbehandlungsmittel zur Bekämpfung von Schuppen umfassend einen Fettsäureester, insbesondere 3-hydroxypropylcaprylat, gegebensfalls in Kombination mit Piroctone Olamine.

Als Antischuppenmittel sind in Wasser oder wässrigen Tensidlösungen lösliche und unlösliche Wirkstoffe bekannt. Diese müssen während eines Reinigungs- und/oder Pflegevorgangs auf behandelten, behaarten Körperoberflächen abgeschieden werden, damit die Antischuppenwirkung eintreten kann.

Üblicherweise werden kationische Polymere als sogenannte Abscheidungshilfsmittel eingesetzt. Sie ermöglichen insbesondere in Kombination mit anionischen Tensiden eine gute Abscheidung von in Wasser oder wässrigen Tensidlösungen unlöslichen Antischuppenwirkstoffen, wie beispielsweise von Zinksalzen.

Zur Stabilisierung von Zinksalzen in Antischuppenshampoos müssen jedoch oftmals Polymere auf Acrylsäurebasis verwendet werden. Von Verbrauchern wird bei der Wahl kosmetischer Produkte zunehmend Wert auf umweltschonende und/oder die Umwelt nicht belastende Inhaltsstoffe (wie Mikroplastik) gelegt, deshalb ist man bestrebt, auf die Verwendung von Stabilisierungspolymeren zu verzichten.

In wässrigen Tensidlösungen gut lösliche Antischuppenwirkstoffe, wie Piroctone Olamine, weisen hingegen den Nachteil auf, dass sie nur schwer und/oder nur in geringen Mengen aus kosmetischen Mitteln auf der Kopfhaut abgeschieden werden, vor allem aus kosmetischen Mitteln, die nach der Anwendung ausgespült werden.

Die der Erfindung zugrundeliegende Aufgabe besteht in der Bereitstellung eines kosmetischen Haarbehandlungsmittels mit Antischuppenwirkung, dessen Leistungsfähigkeit erhöht ist.

Neben einer gesteigerten Antischuppenwirksamkeit ist unter erhöhter Leistungsfähigkeit zudem die schonende Reinigung und/oder Pflege der Kopfhaut und der Haare zu verstehen.

Die der Erfindung zugrundeliegenden Aufgaben werden durch den Gegenstand von Patentanspruch 1 gelöst.

Die Kombination mindestens zweier unterschiedlicher Antischuppenwirkstoffe, umfassend 3-Hydroxypropyloctansäureester (Propandiol Caprylate) und Piroctone Olamine, führte zu Haarbehandlungsmitteln
- mit erhöhter, beschleunigter und/oder länger anhaltender Antischuppenwirksamkeit,
- bei denen die Einstellung der gewünschten Viskosität einen geringeren Anteil oder keine zusätzlichen Verdickungsmittel erforderlich macht,
- mit ausgezeichneten Schaumeigenschaften (im Falle von erfindungsgemäßen Mitteln, die als Haarreinigungsmittel konfektioniert werden),
- mit verbesserten Pflegeeigenschaften, insbesondere in Bezug auf Griff, Elastizität und Geschmeidigkeit der Haare.

Ein erster Gegenstand der Erfindung ist ein Haarbehandlungsmittel mit einem Gehalt an
a) 3-Hydroxypropyloctansäureester (Propandiol Caprylate) und
b) mindestens einen von a) verschiedenen Antischuppenwirkstoff wobei die Antischuppenwirkstoffe a) und b) in einem Gewichtsanteil von bis zu 0,5 Gew.-% am Gesamtgewicht des Haarbehandlungsmittels enthalten sind und wobei der mindestens eine Antischuppenwirkstoff b) Piroctone Olamine enthält.

Als ersten wesentlichen Bestandteil enthalten die erfindungsgemäßen Haarbehandlungsmittel 3-Hydroxypropyloctansäureester (INCI-Bezeichnung: Propanediol Caprylate).

Propanediol Caprylate ist als hochwirksamer Antischuppenwirkstoff bekannt. Es ist ausschließlich aus natürlichen Quellen, beispielsweise durch Hydrolyse von Palmkernöl, anschließender Fermentation und Destillation der Hydrolyseprodukte sowie Veresterung von 1,3-Propandiol mit Caprylsäure erhältlich.

Propanediol Caprylate wirkt gezielt gegen Kopfschuppen verursachende und/oder verstärkende Malassezia-Stämme. Darüber hinaus wurden schaumstabilisierende Eigenschaften von Propanediol Caprylate in üblichen, Tenside enthaltenden Produkten beschrieben.

Die Kombination von Propanediol Caprylate und mindestens einem weiteren Antischuppenwirkstoff enthaltend Piroctone Olamine in kosmetischen Haarbehandlungsmitteln führt zu einer starken Steigerung der Antischuppenwirksamkeit der Mittel, unabhängig davon, ob in Wasser oder wässrigen Tensidlösungen lösliche oder unlösliche Antischuppenwirkstoffe verwendet werden.

Ein weiterer Vorteil ist, dass den Haarbehandlungsmitteln keine oder nur sehr geringe Mengen an freien Salzen und/oder nicht-polymeren Verdickungsmitteln hinzugefügt werden müssen, um die gewünschte Viskosität zu erzielen.

Erfindungsgemäße Haarbehandlungsmittel, die als Reinigungsmittel konfektioniert wurden, weisen zudem ein hohes Schaumvolumen und eine verbesserte Cremigkeit und Haptik des Schaums auf.

Überraschend ist, dass diese Effekte selbst bei geringen Einsatzmengen von Propanediol Caprylate beobachtet werden können.

Propanediol Caprylate ist im Handel erhältlich, beispielsweise von der Firma Symrise unter der Handelsbezeichnung Crinipan^{®} PMC green.

3-Hydroxypropyloctansäureester (INCI-Bezeichnung: Propanediol Caprylate) wird in den erfindungsgemäßen Haarbehandlungsmitteln bevorzugt in einer Menge 0,1 bis 0,4 Gew.-% eingesetzt, wobei sich die Mengenangaben auf das Gesamtgewicht der Haarbehandlungsmittel beziehen.

Als Antischuppenwirkstoff(e) b) enthalten die erfindungsgemäßen Haarbehandlungsmittel vorzugsweise eine oder mehrere Verbindungen aus den folgenden Gruppen:
b1) Zinksalze,
b2) Piroctone Olamine,
b3) Selensulfid,
b4) Salicylsäure,
b5) Climbazol.

Die Antischuppenwirkstoffe b1) bis b5) können in den erfindungsgemäßen Haarbehandlungsmitteln einzeln oder als Mischung in einer Gesamtmenge von 0,1 bis 0,4 Gew.-% eingesetzt werden, wobei sich die Mengenangaben auf das Gesamtgewicht der Haarbehandlungsmittel beziehen.

Vorteilhaft ist, wenn Propanediol Caprylate und der oder die Antischuppenwirkstoff(e) b) in einem Gewichtsverhältnis von 1 : 3 bis 3 : 1 eingesetzt werden.

In einer bevorzugten Ausführungsform enthalten die erfindungsgemäßen Haarbehandlungsmittel 3-Hydroxypropyloctansäureester und den mindestens einen Antischuppenwirkstoff b) in einem Gewichtsverhältnis von 1 : 3 bis 3 : 1.

Unter geeigneten Zinksalzen b1) im Sinne der vorliegenden Erfindung werden vorzugsweise Zinkoxid, Zinkcarbonat und/oder Zinkpyrithion verstanden.

Besonders bevorzugt ist Zinkpyrithion.

Unter den in Wasser oder wässrigen Tensidlösungen unlöslichen Antischuppenwirkstoffen b) sind Zinksalze b1), insbesondere Zinkpyrithion, besonders bevorzugt.

Ein besonders geeigneter in Wasser oder wässrigen Tensidlösungen löslicher Antischuppenwirkstoff b) ist Piroctone Olamine b2).

Die erfindungsgemäße Haarbehandlungsmittel enthalten als Antischuppenwirkstoff b) Piroctone Olamine.

In einer weiteren bevorzugten Ausführungsform enthalten erfindungsgemäße Haarbehandlungsmittel als Antischuppenwirkstoff b) eine Mischung aus Piroctone Olamine und mindestens einem Zinksalz, ausgewählt aus der Gruppe Zinkpyrithion, Zinkoxid und/oder Zinkcarbonat.

Eine weitere bevorzugte Ausführungsform ist dadurch gekennzeichnet, dass erfindungsgemäße Haarbehandlungsmittel 3-Hydroxypropyloctansäureester und Piroctone Olamine in einem Gewichtsverhältnis von 1 : 3 bis 3 : 1 enthalten.

Noch eine weitere bevorzugte Ausführungsform ist dadurch gekennzeichnet, dass erfindungsgemäße Haarbehandlungsmittel 3-Hydroxypropyloctansäureester und Zink Pyrithion in einem Gewichtsverhältnis von 1 : 3 bis 3 : 1 enthalten.

Die tägliche Anwendung einiger handelsüblicher Antischuppenzusammensetzungen (beispielsweise umfassend ausschließlich höhere Mengen an Zinkpyrithion) kann bei täglicher Anwendung - insbesondere bei sensibler Kopfhaut - mitunter zur Austrocknung und im schlimmsten Fall zur Verstärkung von Schuppen führen.

Die erfindungsgemäßen Haarbehandlungsmittel weisen diesen Nachteil nicht auf. Sie können regelmäßig angewendet werden und vermindern, beseitigen, lindern und/oder beugen (Kopf)-Schuppen vor. Der Effekt tritt auch bei geringen Einsatzmengen an Antischuppenwirkstoffen a) und b) auf, beispielsweise in Haarbehandlungsmitteln, die - bezogen auf ihr Gewicht - jeweils 0,05 - 0,4 Gew.-% der Komponenten a) und b) umfassen.

Die Antischuppenwirkstoffe a) und b) sind in den erfindungsgemäßen Haarbehandlungsmitteln in einem Gewichtsanteil von bis zu 0,5 Gew.-%.

Die erfindungsgemäßen Haarbehandlungsmittel enthalten die Wirkstoffe a) und b) bevorzugt in einem kosmetischen Träger. Darunter wird im Rahmen der Erfindung bevorzugt ein wässriger oder ein wässrigalkoholischer Träger verstanden.

Der kosmetische Träger enthält bevorzugt mindestens 70 Gew.-%, besonders bevorzugt mindestens 72,5 Gew.-% und insbesondere bevorzugt mindestens 75 Gew.-% Wasser.

Weiterhin kann der kosmetische Träger 0,01 bis 10 Gew.-%, bevorzugt 0,05 bis 9 Gew.-% und insbesondere 0,10 bis 6 Gew.-% mindestens eines Alkohols enthalten.

Geeignete Alkohole sind beispielsweise Ethanol, Ethyldiglykol, 1-Propanol, 2-Propanol, Isopropanol, 1,2-Propylenglycol, Glycerin, Diglycerin, Triglycerin, 1-Butanol, 2-Butanol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycolen, Sorbitol, Sorbitan, Benzylalkohol, Phenoxyethanol oder Mischungen dieser Alkohole.

Die erfindungsgemäßen Haarbehandlungsmittel können als Rinse-off-Haarreinigungsmittel oder als Leave-on Haarkonditioniermittel konfektioniert werden.

Erfindungsgemäße Rinse-off-Haarreinigungsmittel enthalten neben den Antischuppenwirkstoffen a) und b) vorzugsweise mindestens ein waschaktives Tensid, ausgewählt aus anionischen, amphoteren, zwitterionischen und/oder nichtionischen Tensiden.

Milde und gute Schaumeigenschaften erfindungsgemäßer Rinse-off-Haarreinigungsmittel können durch die sorgfältige Auswahl der Tensidmengen und/oder der Tensidtypen gesteuert werden.

Bevorzugt enthalten erfindungsgemäße Rinse-off-Haarreinigungsmittel mindestens ein anionisches Tensid, welches für die Erzeugung zufriedenstellender Schaummengen und Schaumeigenschaften verantwortlich ist. Für die Erzielung einer optimalen Balance zwischen Milde und Schaumeigenschaften ist eine Mischung aus mindestens einem anionischen Tensid und mindestens einem milden Co-Tensid besonders bevorzugt. Das milde Co-Tensid kann vorzugsweise aus amphoteren und/oder zwitterionischen und/oder nichtionischen Tensiden ausgewählt werden.

In einer bevorzugten Ausführungsform enthalten erfindungsgemäße Haarbehandlungsmittel mindestens ein anionisches, amphoteres, zwitterionisches, nichtionisches und/oder kationisches Tensid in einem Gewichtsanteil von bis zu 15 Gew.-% am Gesamtgewicht des Haarbehandlungsmittels.

In einer weiteren bevorzugten Ausführungsform werden erfindungsgemäße Haarbehandlungsmittel als Rinse-off-Haarreinigungsmittel konfektioniert und enthalten mindestens ein anionisches Tensid in einem Gewichtsanteil von bis zu 12,5 Gew.-% am Gesamtgewicht des Haarbehandlungsmittels.

In einer weiteren bevorzugten Ausführungsform werden erfindungsgemäße Haarbehandlungsmittel als Rinse-off-Haarreinigungsmittel konfektioniert und enthalten
(i) mindestens ein anionisches Tensid in einem Gewichtsanteil von 1 bis 10 Gew.-% am Gesamtgewicht des Haarbehandlungsmittels und
(ii) mindestens ein amphoteres und/oder zwitterionisches Tensid in einem Gewichtsanteil von 0,1 bis 5 Gew.-% am Gesamtgewicht des Haarbehandlungsmittels und/oder
(iii) mindestens ein nichtionisches Tensid in einem Gewichtsanteil von 0,1 bis 5 Gew.-% am Gesamtgewicht des Haarbehandlungsmittels.

In einer weiteren bevorzugten Ausführungsform werden erfindungsgemäße Haarbehandlungsmittel als Leave-on- oder Rinse-off-Haarpflegemittel konfektioniert und enthalten mindestens ein kationisches Tensid in einem Gewichtsanteil von bis zu 5 Gew.-% am Gesamtgewicht des Haarbehandlungsmittels.

Geeignete anionische Tenside im Sinne der vorliegenden Erfindung sind:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe (Sarcosinat-Tenside),
- Acyltauride mit 8 bis 24 C-Atomen in der Acylgruppe (Taurat-Tenside),
- Acylisethionate mit 8 bis 24 C-Atomen in der Acylgruppe (Isethionat-Tenside),
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen (Sulfosuccinat-Tenside),
- Alpha-Olefinsulfonate mit 8 bis 24 C-Atomen (alpha-Olefinsulfonat-Tenside),
- Alkylsulfate und/oder Alkylethersulfatsalze der Formel R-(OCH₂-CH₂)ₙ-O-SO₃X, in der R bevorzugt eine geradkettige oder verzweigte, gesättigte oder ungesättigte Alkylgruppe mit 8 bis 30 C-Atomen, x die Zahl 0 oder 1 bis 12 und X ein Alkali-, Erdalkali-, Ammonium- oder Alkanolaminion bedeutet,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen, und/oder
- Alkyl- und/oder Alkenyletherphosphate der Formel 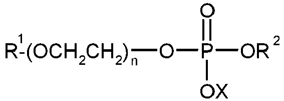
in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder die Gruppe -NR³R⁴R⁵R⁶ steht, mit R³ bis R⁶ unabhängig voneinander stehend für einen C₁ bis C₄ -Kohlenwasserstoffrest.

Besonders bevorzugt sind Alkylsulfat- und/oder Alkylethersulfatsalze, (Salze von) Ethercarbonsäuren, Sarcosinaten, Isethionaten, Tauraten, Sulfosuccinaten und/oder Alpha-Olefinsulfonaten, insbesondere Alkylsulfat- und/oder Alkylethersulfatsalze.

Geeignete amphotere und/oder zwitterionische Tenside im Sinne der vorliegenden Erfindung sind insbesondere milde Tenside mit hervorragenden Schaumeigenschaften.

Darunter werden vorzugsweise oberflächenaktive Verbindungen verstanden, die ausgewählt sind aus der Gruppe umfassend Alkylbetaine, Alkylamidoalkylbetaine, Alkylamphoacetate, Alkylamphodiacetate, Alkylamphopropionate, Alkylamphodipropionate, Alkylsultaine, Alkylhydroxysultaine, Alkylaminoxide, Alkylamphoglycinate, Alkyliminodiacetate, Alkyliminodipropionate, Alkylamphopropylsulfonate, Alkylamphocarboxyglycinate und Alkylamphocarboxypropionate.

Geeignete Alkylbetaine und/oder Alkylamidopropylbetaine umfassen vorzugsweise C₄-C₂₄-, mehr bevorzugt C₆-C₁₈-, besonders bevorzugt C₈-C₁₄-Alkylketten, die linear oder verzweigt sein können, wobei lineare bevorzugt sind. Besonders bevorzugte Alkylbetaine oder Alkylamidopropylbetaine sind ausgewählt aus der Gruppe Lauryl Betaine, Coco-Betaine, Behenyl Betaine, Capryl/Capramidopropyl Betaine, Cetyl Betaine, Cocamidoethyl Betaine, Cocamidopropyl Betaine, Coco/Oleamidopropyl Betaine, Decyl Betaine, Dimer Dilinoleamidopropyl Dibetaine, Hydrogenated Tallow Betaine, Hydroxylauryl/Hydroxymyristyl Betaine, Isostearamidopropyl Betaine, Lauramidopropyl Betaine, Lauryl Betaine, Myristyl Betaine, Oleamidopropyl Betaine, Oleyl Betaine, Palmitamidopropyl Betaine, Ricinoleamidopropyl Betaine, Stearamidopropyl Betaine, Stearyl Betaine, Tallowamidopropyl Betaine, Tallow Betaine, Tallow Dihydroxyethyl Betaine, Undecylenamidopropyl Betaine, Sunfloweramidopropyl Betaine, Cetyl Betaine, Lauryl Betaine.

Ganz besonders bevorzugt ist Cocamidopropyl Betaine.

Besonders geeignete Alkylamphoacetate, Alkylamphodiacetate, Alkylamphopropionate oder Alkylamphodipropionate sind ausgewählt aus der Gruppe Cocobetainamido Amphopropionate, DEA-Cocoamphodipropionate, Disodium Caproamphodiacetate, Disodium Caproamphodipropionate, Disodium Capryloamphodiacetate, Disodium Capryloamphodipropionate, Disodium Cocoamphodiacetate, Disodium Cocoamphodipropionate, Disodium Isostearoamphodiacetate, Disodium Isostearoamphodipropionate, Disodium Laureth-5 Carboxyamphodiacetate, Disodium Lauroamphodiacetate, Disodium Lauroamphodipropionate, Disodium Oleoamphodipropionate, Disodium PPG-2-Isodeceth-7 Carboxyamphodiacetate, Disodium Stearoamphodiacetate, Disodium Tallowamphodiacetate, Sodium Caproamphopropionate, Sodium Capryloamphoacetate, Sodium Capryloamphopropionate, Sodium Cocoamphoacetate, Sodium Cocoamphopropionate, Sodium Isostearoamphoacetate, Sodium Isostearoamphopropionate, Sodium Lauroamphoacetate, Sodium Lauroamphopropionate, Sodium Myristoamphoacetate, Sodium Oleoamphoacetate, Sodium Oleoamphopropionate, Sodium Stearoamphoacetate, Sodium Stearoamphopropionate, Sodium Tallamphopropionate, Sodium Tallowamphoacetate, Sodium Undecylenoamphoacetate und Sodium Undecylenoamphopropionate.

Ganz besonders bevorzugt sind Sodium Cocoamphoacetate und Disodium Cocooamphodiacetate. Besonders geeignete Alkylsultaine oder Alkylhydroxysultaine sind ausgewählt aus der Gruppe Capryl Sultaine, Cocamidopropyl Hydroxysultaine, Coco-Hydroxysultaine, Coco-Sultaine, Erucamidopropyl Hydroxysultaine, Lauramidopropyl Hydroxysultaine, Lauryl Hydroxysultaine, Lauryl Sultaine, Myristamidopropyl Hydroxysultaine, Oleamidopropyl Hydroxysultaine, Tallowamidopropyl Hydroxysultaine.

Besonders geeignete Alkylaminoxide sind ausgewählt aus der Gruppe Behenamine Oxide, Cocamidopropylamine Oxide, Cocamine Oxide, Decylamine Oxide, Decyltetradecylamine Oxide, Dihydroxyethyl C8-10 Alkoxypropylamine Oxide, Dihydroxyethyl C9-11 Alkoxypropylamine Oxide, Dihydroxyethyl C12-15 Alkoxypropylamine Oxide, Dihydroxyethyl Cocamine Oxide, Dihydroxyethyl Lauramine Oxide, Dihydroxyethyl Stearamine Oxide, Dihydroxyethyl Tallowamine Oxide, Hydrogenated Tallowamine Oxide, Isostearamidopropylamine Oxide, Lauramidopropylamine Oxide, Lauramine Oxide, Myristamidopropylamine Oxide, Myristamine Oxide, Palmitamidopropylamine Oxide, Palmitamine Oxide, PEG-3 Lauramine Oxide, Stearamidopropylamine Oxide, Stearamine Oxide, Tallowamidopropylamine Oxide, Tallowamine Oxide, Undecylenamidopropylamine Oxide.

Besonders geeignete Alkylamphoglycinate sind ausgewählt aus der Gruppe Caproamphoglycinate, Capryloamphoglycinate, Cocoamphoglycinate, Isostearoamphoglycinate, Lauroamphoglycinate, Myristoamphoglycinate, Oleoamphoglycinate, Stearoamphoglycinate, Tallowamphoglycinate, Undecylenoamphoglycinate.

Besonders geeignete Alkyliminodiacetate oder Alkyliminodipropionate sind ausgewählt aus der Gruppe Disodium Cocaminopropyl Iminodiacetate, Disodium Hydroxyethyliminodiacetate, Disodium Lauriminodiacetate, Disodium Lauriminodipropionate, Disodium Steariminodipropionate, Disodium Tallowiminodipropionate, Sodium C12-15 Alkoxypropyl Iminodipropionate, Sodium Cocoiminodiacetate, Sodium Lauriminodipropionate.

Besonders geeignete Alkylamphopropylsulfonate sind ausgewählt aus Sodium Cocoamphohydroxypropylsulfonate, Sodium Lauroamphohydroxypropylsulfonate, Sodium Oleoamphohydroxypropylsulfonate, Sodium Stearoamphohydroxypropylsulfonate, Disodium Lauriminobishydroxypropylsulfonate, Disodium Cocoamphocarboxyethylhydroxypropylsulfonate, Sodium Caproamphohydroxypropylsulfonate, Sodium Capryloamphohydroxypropylsulfonate.

Erfindungsgemäße Haarbehandlungsmittel, die als Haarreinigungsmittel konfektioniert werden, enthalten als amphotere und/oder zwitterionische Tenside vorzugsweise Alkylamidoalkylbetaine, Alkylamphoacetate und/oder Alkylamphodiacetate.

Geeignete nichtionische Tenside für die Verwendung als Co-Tensid sind beispielsweise
- Aminoxide, die ausgewählt sein können aus Verbindungen der allgemeinen Formeln (I) oder (II) 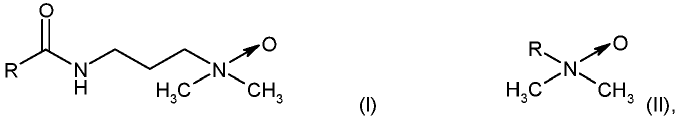
   in denen R jeweils für einen geradkettigen oder verzweigten, gesättigten oder ein- oder mehrfach ungesättigten Alkyl- oder Alkenylrest mit 6 bis 24 Kohlenstoffatomen, bevorzugt mit 8 bis 18 Kohlenstoffatomen steht.
   Insbesondere bevorzugt sind die unter den INCI-Bezeichnungen Cocamine Oxide, Lauramine Oxide und/oder Cocamidopropylaminoxid bekannten und im Handel von verschiedenen Anbietern erhältlichen Tenside der zuvor genannten Formel (I) oder (II).
- Fettsäurealkanolamide der nachfolgenden allgemeinen Formel, 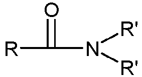 in der R bevorzugt einen linearen oder verzweigten, gesättigten oder ungesättigten Alkyl- oder Alkenylrest mit 8 bis 24 Kohlenstoffatomen bedeutet und die Reste R' für Wasserstoff oder für die Gruppe -(CH₂)ₙOH stehen, in der n die Zahlen 2 oder 3 bedeutet, mit der Maßgabe, dass mindestens einer der Reste R' für den zuvor genannten Rest -(CH₂)ₙOH steht,
- Gemische aus Alkyl-(oligo)-glucosiden und Fettalkoholen zum Beispiel das im Handel erhältliche Produkt Montanov^{®}68,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden,
- Alkyl(oligo)glycoside. Geeignete Alkyl(oligo)glycoside können ausgewählt sein aus Verbindungen der allgemeinen Formel der RO-[G]ₓ, in denen sich [G] bevorzugt von Aldosen und/oder Ketosen mit 5-6 Kohlenstoffatomen, vorzugsweise von Glucose ableitet.
Die Indexzahl x steht für den Oligomerisierungsgrad (DP), d.h. für die Verteilung der Mono- und Oligoglycoside. Die Indexzahl x weist vorzugsweise einen Wert im Bereich von 1 bis 10, besonders bevorzugt im Bereich von 1 bis 3 auf, wobei es sich dabei um keine ganze Zahl, sondern um eine gebrochene Zahl handeln kann, die analytisch ermittelt werden kann.

Besonders bevorzugte Alkyl(oligo)glycoside weisen einen Oligomerisierungsgrad zwischen 1,2 und 1,5 auf. Der Rest R steht bevorzugt für mindestens einen Alkyl- und/oder Alkenylrest mit 4 bis 24 C-Atomen. Insbesondere bevorzugte Alkyl(oligo)glycoside sind die unter den INCI-Bezeichnungen Caprylyl/Capryl Glucoside, Decyl Glucoside, Lauryl Glucoside und Coco Glucoside bekannten Verbindungen.

Besonders bevorzugte nichtionische Tenside, die in erfindungsgemäßen (Haarreinigungs)mitteln als Co-Tensid enthalten sein können, sind Fettsäurealkanolamide und/oder Alkyl(oligo)glucoside. Insbesondere bevorzugt sind Fettsäurealkanolamide.

Geeignete kationische Tenside im Sinne der vorliegenden Erfindung sind:
- quartäre Ammoniumverbindungen, beispielsweise ausgewählt aus Verbindungen der nachfolgenden Formel 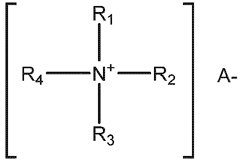 in der
   - die Reste R₁, R₂, R₃ und R₄ jeweils unabhängig voneinander für Wasserstoff, eine Methylgruppe, eine Phenylgruppe, eine Benzylgruppe oder für einen gesättigten, verzweigten oder unverzweigten Alkylrest mit einer Kettenlänge von 8 bis 30 Kohlenstoffatomen stehen, welcher gegebenenfalls mit einer oder mehreren Hydroxygruppen substituiert sein kann,
   - mit der Maßgabe, dass mindestens einer der Reste R₁, R₂, R₃ und R₄ nicht für Wasserstoff steht, und
   - für ein physiologisch verträgliches Anion steht, beispielsweise für ein Halogenid wie Chlorid oder Bromid sowie für Methosulfate.
   Beispiele für bevorzugte quartäre Ammoniumverbindungen der zuvor genannten Formel sind Lauryltrimehtylammoniumchlorid, Cetyltrimethylammoniumchlorid, Cetyltrimethylammoniumbromid, Cetyltrimethylammonium-methosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.
   Insbesondere bevorzugt sind Cetyltrimethylammoniumchlorid und/oder Behenyltrimethylammoniumchlorid.
- Esterquats, beispielsweise ausgewählt aus Verbindungen der nachfolgenden Formel 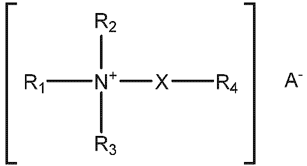
   - worin die Reste R₁, R₂ und R₃ jeweils unabhängig voneinander gleich oder verschieden sein und die folgende Bedeutung haben können:
   - einen verzweigten oder unverzweigten Alkylrest mit 1 bis 4 Kohlenstoffatomen, welcher mindestens eine Hydroxylgruppe enthalten kann, oder
   - einen gesättigten oder ungesättigten, verzweigten oder unverzweigten oder einen cyclischen gesättigten oder ungesättigten Alkylrest mit 6 bis 30 Kohlenstoffatomen, welcher mindestens eine Hydroxylgruppe enthalten kann, oder
   - einen Aryl- oder Alkylarylrest, beispielsweise Phenyl oder Benzyl,
   - den Rest (-X-R₄), mit der Maßgabe, dass höchstens 2 der Reste R₁, R₂ und R₃ für diesen Rest stehen können, wobei
   - X für:
      1) -(CH₂)ₙ- mit n = 1 bis 20 steht, vorzugsweise n = 1 bis 10 und besonders bevorzugt n = 1 bis 5, oder
      2) -(CH₂-CHR₅-O)ₙ- mit n = 1 bis 200 steht, vorzugsweise 1 bis 100, besonders bevorzugt 1 bis 50, und insbesondere 1 bis 20, und R₅ für Wasserstoff, eine Methyl- oder Ethylgruppe steht,
      3) eine Hydroxyalkylgruppe mit ein bis vier Kohlenstoffatomen steht, welche verzweigt oder unverzweigt sein kann, und welche mindestens eine und höchstens 3 Hydroxygruppen enthält. Beispiele sind: -CH₂OH, -CH₂CH₂OH, -CHOHCHOH, - CH₂CHOHCH₃, -CH(CH₂OH)₂, -COH(CH₂OH)₂,
      4) -CH₂CHOHCH₂OH, -CH₂CH₂CH₂OH und Hydroxybutylreste, und
   - R₄ für:
      1) die Gruppe R₆-O-CO- steht, worin R₆ für einen gesättigten oder ungesättigten, verzweigten oder unverzweigten oder einen cyclischen gesättigten oder ungesättigten Alkylrest mit 6 bis 30 Kohlenstoffatomen steht, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
      2) die Gruppe R₇-CO-, worin R₇ für einen gesättigten oder ungesättigten, verzweigten oder unverzweigten oder einen cyclischen gesättigten oder ungesättigten Alkylrest mit 6 bis 30 Kohlenstoffatomen steht, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, und
   - A für ein physiologisch verträgliches organisches oder anorganisches Anion steht, beispielsweise für ein Halogenidion wie Chlorid, Bromid, Iodid, ein Sulfation der Formel RSO₃⁻, worin R die Bedeutung von gesättigtem oder ungesättigtem Alkylresten mit 1 bis 4 Kohlenstoffatomen hat, oder für ein organisches Säureanion wie beispielsweise für Maleat, Fumarat, Oxalat, Tartrat, Citrat, Lactat oder Acetat steht.
- Esterquats, beispielsweise ausgewählt aus Verbindungen der nachfolgenden Formel 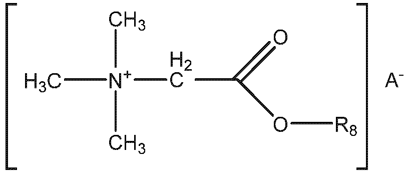
   worin R₈ in seiner Bedeutung der Bedeutung von R₇ entspricht und worin A⁻ dieselbe Bedeutung hat wie zuvor beschrieben.
   Besonders bevorzugt sind Esterquats, welche gemäß der INCI-Nomenklatur als Distearoylethyl Hydroxyethylmonium Methosulfate, Dicocoylethyl Hydroxyethylmonium Methosulfate, Dipalmitoylethyldimonium Chloride, Behenoyl PG Trimonium Chloride bezeichnet werden.

Neben den zuvor genannten wesentlichen und fakultativen Bestandteilen können die erfindungsgemäßen Haarbehandlungsmittel zur Steigerung ihrer pflegenden Eigenschaften weitere haarkonditionierende Wirkstoffe enthalten, solange diese das Schaumvermögen und die Stabilität der Haarbehandlungsmittel nicht negativ beeinträchtigen.

Unter besonders geeigneten haarkonditionierenden Wirkstoffen im Sinne der vorliegenden Erfindung sind vorzugsweise
- kationische Polymere,
- Öl-, Fett- und/oder Wachskomponenten,
- Proteinhydrolysate,
- Pflanzenextrakte zu verstehen.

In einer bevorzugten Ausführungsform enthalten erfindungsgemäße Haarbehandlungsmittel mindestens ein kationisches Polymer in einem Gewichtsanteil von 0,01 bis 3 Gew.-% am Gesamtgewicht des Haarbehandlungsmittels.

In einer weiteren bevorzugten Ausführungsform enthalten erfindungsgemäße Haarbehandlungsmittel mindestens eine Öl-, Fett- oder Wachskomponente in einem Gewichtsanteil von 0,01 bis 5 Gew.-% am Gesamtgewicht des Haarbehandlungsmittels.

In einer weiteren bevorzugten Ausführungsform enthalten erfindungsgemäße Haarbehandlungsmittel mindestens ein Proteinhydrolysat in einem Gewichtsanteil von 0,01 bis 3 Gew.-% am Gesamtgewicht des Haarbehandlungsmittels und/oder mindestens einen Pflanzenextrakt in einem Gewichtsanteil von 0,01 bis 3 Gew.-% am Gesamtgewicht des Haarbehandlungsmittels.

Geeignete kationische Polymere im Sinne der vorliegenden Erfindung sind beispielsweise:
- quaternisierte Cellulosepolymere, vor allem Polyquaternium-10, wie sie unter den Bezeichnungen Celquat^{®} und Polymer JR^{®} im Handel erhältlich sind,
- hydrophob modifizierte kationische Cellulosederivate, beispielsweise die unter dem Handelsnamen SoftCat^{®} vertriebenen kationischen Polymere,
- kationische Alkylpolyglycoside,
- kationiserter Honig, beispielsweise das Handelsprodukt Honeyquat^{®} 50,
- kationische Galactomannan-Derivate, insbesondere kationische Guar- und/oder Cassia-Polymere, wie die unter den Handelsnamen Jaguar^{®}, N-Hance^{®}, Polycare^{®}, Clearhance^{®} erhältlichen Produkte. Insbesondere geeignet sind: Jaguar^{®} C-162, Jaguar^{®} C500, Jaguar^{®} Styl 100, Jaguar^{®} Excel, N-Hance^{®} 3196, N-Hance^{®} HPCG 1000 und/oder Polycare^{®} Split Therapy,
- kationische Stärke-Derivate, wie sie beispielsweise unter der Bezeichnung Mirustyle^{®} im Handel erhältlich sind,
- kationische Inulin-Polxmere,
- polymere Dimethyldiallylammoniumsalze und deren Copolymere mit Estern und Amiden von Acrylsäure und Methacrylsäure, vor allem Polyquaternium-6 und Polyquaternium-7. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium-2, Polyquaternium-17, Polyquaternium-18, Polyquaternium-22, Polyquaternium-24, Polyquaternium-27, Polyquaternium-32, Polyquaternium-37, Polyquaternium-67, Polyquaternium-74 und Polyquaternium-89 bekannten Polymere.

Bevorzugt sind kationische Polymere natürlichen Ursprungs wie quaternisierte Cellulosepolymere, hydrophob modifizierte kationische Cellulosederivate und/oder kationische Guarderivate.

Besonders bevorzugt sind die unter den INCI-Bezeichnungen Guar Hydroxypropyltrimonium Chloride, Polyquaternium-10, Polyquaternium-67 und/oder Hydroxypropyltrimonium Hydrolyzed Corn Starch bekannten kationischen Polymere; insbesondere bevorzugt sind Guar Hydroxypropyltrimonium Chloride und Polyquaternium-10.

Geeignete Öl-, Fett- und/oder Wachskomponenten im Sinne der vorliegenden Erfindung sind:
- mineralische Öle wie Paraffin- und Isoparaffinöle und synthetische Kohlenwasserstoffe. Ein Beispiel für einen einsetzbaren Kohlenwasserstoff ist beispielsweise das als Handelsprodukt erhältliche 1,3-Di-(2-ethylhexyl)-cyclohexan (Cetiol^{®} S).
- Dialkylether, vorzugsweise Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel unter der Bezeichnung Cetiol^{®} OE erhältlich ist.
- Fettalkohole können gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂-Kohlenstoffatomen eingesetzt werden. Beispiele dafür sind Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.
- Esteröle wie die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-O18 (INCI-Bezeichnung: Glyceryl Oleate), Monomuls^{®} 90-L12 (INCI-Bezeichnung: Glyceryl Laurate), Cetiol^{®} HE (INCI-Bezeichnung: PEG-7 Glyceryl Cocoate) oder Cutina^{®} MD (INCI-Bezeichnung: Glyceryl Stearate).
- natürliche Öle und/oder Buttern wie beispielsweise Kokosöl, (süßes) Mandelöl, Walnussöl, Pfirsichkernöl, Aprikosenkernöl, Avocadoöl, Teebaumöl (Tea Tree Oil), Sojaöl, Baumwollsaatöl, Sesamöl, Sonnenblumenöl, Tsubakiöl, Nachtkerzenöl, Reiskleieöl, Palmöl, Palmkernöl, Mangokernöl, Cranberryöl, Sanddornöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Marulaöl, Granatapfelkernöl, Paradiesnussöl, Passionsfruchtkernöl, Hagebuttenkernöl, Haselnussöl, Hanföl, Kaffeeöl, Rizinusöl, Safloröl, Maisöl, Olivenöl, Rapsöl, Canolaöl, Sasanquaöl, Jojobaöl, Rambutanöl, Sheabutter, Mangobutter, Murumurubutter, Kakaobutter, Aprikosenkernbutter, Mafurabutter, Bacuributter, Tucumabutter, Ucuubabutter und/oder Cupuacubutter.
- Wachse, beispielsweise natürliche Wachse wie Carnaubawachs, Candelillawachs und/oder Jojobaöl sowie tierische Wachse wie Bienenwachs, Wollwachs, Walrat und/oder Bürzeldrüsenfett und/oder synthetische Mineralwachse wie Hartparaffin, Ceresin, Ozokerit, Esterwachse wie Polyethylenglycol- oder Polyethylenglycolesterwachse und/oder gehärtete Pflanzenöle (insbesondere Hydrogenated Castor Oil).

Unter geeigneten Proteinhydrolysaten sind Produktgemische zu verstehen, die durch sauer, basisch oder enzymatisch katalysierten Abbau von Proteinen (Eiweißen) erhalten werden können.

Es können Proteinhydrolysate pflanzlichen, tierischen und/oder marinen Ursprungs eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Bevorzugt sind Proteinhydrolysate pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Einsetzbar sind auch kationisierte Proteinhydrolysate, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder von biotechnologisch gewonnenen Proteinhydrolysaten, stammen kann. Die den kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die kationischen Proteinhydrolysate und -derivate seien die unter den INCI - Bezeichnungen bekannten und im Handel erhältlichen Produkte genannt: Cocodimonium Hydroxypropyl Hydrolyzed Collagen, Cocodimopnium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed keratin, Hydroxypropyltrimonium Hydrolyzed Rice Bran Protein, Hydroxyproypltrimonium Hydrolyzed Silk, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Silk, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Silk, Quaternium-79 Hydrolyzed Soy Protein, Quaternium-79 Hydrolyzed Wheat Protein.

Geeignete Pflanzenextrakte sind vor allem die Extrakte aus grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Dattelpalme, Zimtbaum, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräserwie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea angustifolia DC, Echinacea paradoxa (Norton), Echinacea simulata, E. atrorubens, E. tennesiensis, Echinacea strigosa (Mc Gregor), Echinacea laevigata, Echinacea purpurea (L.) Moench und Echinacea pallida (Nutt), aller Arten von Algen, Korallenmoos und Seetang (wie beispielsweise Macrocystis Pyrifera Extrakt), aller Arten von Wein sowie Perikarp von Litchie chinensis. Geeignete Extrakte können aus den Früchten, Samen, Blüten, Wurzeln, Blättern und/oder Rinden der o.g. Pflanzen oder aus den gesamten Pflanzen (Algen, Seetang) gewonnen werden.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch. Geeignete Extraktionsmittel sind üblicherweise Wasser und/oder Alkohole.

Weitere Wirk-, Hilfs- und Zusatzstoffe, die in den erfindungsgemäßen Haarbehandlungsmitteln bevorzugt enthalten sein können, sind beispielsweise:
- Parfums,
- UV-Filter,
- (natürliche) Farbstoffe zum Anfärben des Mittels,
- Wirkstoffe wie Bisabolol und/oder Allantoin,
- Komplexbildner wie EDTA, NTA, β-Alanindiessigsäure und Phosphonsäuren,
- Ceramide. Unter Ceramiden werden N-Acylsphingosin (Fettsäure des Sphingosins) oder synthetische Analoga solcher Lipide (sogenannte Pseudo-Ceramide) verstanden,
- Antioxidantien,
- Konservierungsmittel, wie beispielsweise Natriumbenzoat oder Salicylsäure,
- pH-Stellmittel wie Zitronensäure und/oder Milchsäure,
- Viskositätsregler wie Salze (NaCl).

Ein zweiter Gegenstand der Erfindung ist die Verwendung einer Wirkstoffkombination umfassend 3-Hydroxypropyloctansäureester a) und mindestens einen von a) verschiedenen Antischuppenwirkstoff in Haarbehandlungsmitteln zur
a. Erzielung einer verbesserten Antischuppenwirksamkeit,
b. Optimierung der Viskositätseinstellung,
c. Stabilisierung der Schaumeigenschaften,
d. Verbesserung des Haargriffs,
e. Verbesserung der Elastizität der Haare,
f. Verbesserung der Geschmeidigkeit der Haare.

Ein dritter Gegenstand der Erfindung ist die Verwendung des erfindungsgemäßen Haarbehandlungsmittels zur Reinigung und Pflege menschlicher Haare sowie zur Prophylaxe, Verminderung, Beseitigung und Linderung von Schuppen auf behaarten Körperoberflächen, insbesondere als Antischuppenshampoo.

Bezüglich weiterer bevorzugter Ausführungsformen der erfindungsgemäßen Verwendung gilt das zu den erfindungsgemäßen Zusammensetzungen Gesagte.

Die folgenden Beispiele sollen den Gegenstand der vorliegenden Erfindung erläutern, ohne ihn darauf zu beschränken.

### Beispiele:

Es wurden die folgenden Haarbehandlungsmittel hergestellt (die Mengenangaben beziehen sich auf [Gew.-%]):

### a) Rinse-off-Zusammensetzungen

| | **1** | **2** | **3** | **4** |
|---|---|---|---|---|
| Propanediol Caprylate | 0,01 - 4 | 0,075 - 0,75 | 0,1 - 0,4 | 0,1 - 0,4 |
| Piroctone Olamine | 0,01 - 2 | 0,075 - 0,75 | | 0,1 - 0,4 |
| Zink Pyrithion | 0,01 - 2 | | 0,1 - 0,4 | |
| anionisches Tensid | 0,5 - 12,5 | 0,5 - 12,5 | | |
| Sodium Laureth Sulfate | | | 1 - 10 | 1 - 10 |
| amphoteres Tensid | 0,1 - 5 | | | |
| Disodium Cocoamphodiacetate | | 0,1 - 5 | | 0,1 - 5 |
| Cocoamidopropylbetaine | | | 0,1 - 5 | 0,1 - 5 |
| nichtionisches Tensid | 0,1 - 5 | | | |
| Cocamide MEA | | | | 0,1 - 1 |
| Öl-, Fett- oder Wachs | 0,01 - 5 | | | |
| Jojobaöl | | | | 0,01 - 1 |
| Aprikosenkernöl | | | 0,01 - 1 | |
| kationisches Polymer | 0,01 - 3 | | | 0,1 - 0,6 |
| Guar Hydroxypropyltrimonium Chloride | | 0,01 - 1 | | 0,01 - 1 |
| Polyquaternium-10 | | | 0,01 - 1 | |
| Zitronensäure | 0,1 - 0,5 | 0,1 - 0,5 | 0,1 - 0,5 | 0,1 - 0,5 |
| Natriumchlorid | 0 - 0,5 | 0 - 0,5 | 0,1 - 0,5 | 0,1 - 0,3 |
| Konservierungsmittel | q.s. | q.s. | q.s. | q.s. |
| Wasser | ad 100 | ad 100 | ad 100 | Wasser |
| pH-Wert | 4,5 - 5,0 | 4,5 - 5,0 | 4,8 ± 0,2 | pH-Wert |

### b) Haarpflegemittel

| | **5** | **6** | **7** | **8** |
|---|---|---|---|---|
| Propanediol Caprylate | 0,01 - **4** | 0,075 - 0,75 | 0,1 - 0,4 | 0,1 - 0,4 |
| Piroctone Olamine | 0,01 - 2 | 0,075 - 0,75 | | 0,1 - 0,4 |
| Zink Pyrithion | 0,01 - 2 | | 0,1 - 0,4 | |
| Esterquat | 0,1 -3 | 0,1 -3 | 0,1 -3 | 0,1 -3 |
| Cetrimonium Cloride | 0,1-3 | 0,1-3 | 0,1-3 | 0,1-3 |
| Cetearyl Alcohol | 0,5-10 | 0,5-10 | 0,5-10 | 0,5-10 |
| Glycerin | 0,01-8 | 0,01-8 | 0,01-8 | 0,01-8 |
| Silikonöl | 0-10 | 0-10 | 0-10 | 0-10 |
| Öl-, Fett- oder Wachs | 0,01 - 5 | 0,01 - 5 | | |
| Jojobaöl | | | | 0,01 - 1 |
| Aprikosenkernöl | | | 0,01 - 1 | |
| Guar Hydroxypropyltrimonium Chloride | | 0,01 - 1 | | 0,01 - 1 |
| Polyquaternium-10 | | | 0,01 - 1 | |
| Zitronensäure | 0,1 - 0,5 | 0,1 - 0,5 | 0,1 - 0,5 | 0,1 - 0,5 |
| Konservierungsmittel | q.s. | q.s. | q.s. | q.s. |
| Wasser | ad 100 | ad 100 | ad 100 | ad 100 |
| pH-Wert | 2,5-4,8 | 2,5-4,8 | 2,5-4,8 | 2,5-4,8 |

### c) Beurteilung

Die beiden Shampoos A (erfindungsgemäß) und B in der nachfolgenden Tabelle wurden im Haarstudio an drei Modellen getestet und von Experten beurteilt.

| | **A** | **B** |
|---|---|---|
| Propanediol Caprylate | 0,25 | |
| Piroctone Olamine | 0,25 | 0,5 |
| Sodium Laureth Sulfate | 9,1 | 9,1 |
| Cocoamidopropylbetaine | 3,0 | 3,0 |
| Guar Hydroxypropyltrimonium Chloride | 0,25 | 0,25 |
| Natriumchlorid | 0,25 | 0,25 |
| Konservierungsmittel, pH-Stellmittel | q.s. | q.s. |
| Wasser | ad 100 | ad 100 |
| pH-Wert | 4,5 - 5,0 | 4,5 - 5,0 |

Neben einer sehr guten Antischuppenwirkung zeigt Shampoo A gegenüber Shampoo B insbesondere eine schnellere Schaumentwicklung (Blitzschaum - flash foam) sowie ein höheres Schaumvolumen.

## Patentansprüche

1. Haarbehandlungsmittel, enthaltend
a) 3-Hydroxypropyloctansäureester (Propandiol Caprylate) und
b) mindestens einen von a) verschiedenen Antischuppenwirkstoff
wobei die Antischuppenwirkstoffe a) und b) in einem Gewichtsanteil von bis zu 0,5 Gew.-% am Gesamtgewicht des Haarbehandlungsmittels enthalten sind und
wobei der mindestens eine Antischuppenwirkstoff b) Piroctone Olamine enthält.

2. Haarbehandlungsmittel nach Anspruch 1, enthaltend als Antischuppenwirkstoff b) eine Mischung aus Piroctone Olamine und mindestens einem Zinksalz, ausgewählt aus der Gruppe Zinkpyrithion, Zinkoxid und/oder Zinkcarbonat.

3. Haarbehandlungsmittel nach einem der Ansprüche 1 oder 2, enthaltend 3-Hydroxypropyloctansäureester und den Antischuppenwirkstoff b) in einem Gewichtsverhältnis von 1 : 3 bis 3 : 1.

4. Haarbehandlungsmittel nach einem der vorhergehenden Ansprüche, enthaltend mindestens ein anionisches, amphoteres, zwitterionisches, nichtionisches und/oder kationisches Tensid in einem Gewichtsanteil von bis zu 15 Gew.-% am Gesamtgewicht des Haarbehandlungsmittels.

5. Haarbehandlungsmittel nach einem der vorhergehenden Ansprüche, enthaltend mindestens ein anionisches Tensid in einem Gewichtsanteil von bis zu 12,5 Gew.-% am Gesamtgewicht des Haarbehandlungsmittels.

6. Haarbehandlungsmittel nach einem der vorhergehenden Ansprüche, enthaltend
(i) mindestens ein anionisches Tensid in einem Gewichtsanteil von 1 bis 10 Gew.-% am Gesamtgewicht des Haarbehandlungsmittels und
(ii) mindestens ein amphoteres und/oder zwitterionisches Tensid in einem Gewichtsanteil von 0,1 bis 5 Gew.-% am Gesamtgewicht des Haarbehandlungsmittels und/oder
(iii) mindestens ein nichtionisches Tensid in einem Gewichtsanteil von 0,1 bis 5 Gew.-% am Gesamtgewicht des Haarbehandlungsmittels.

7. Haarbehandlungsmittel nach einem der vorhergehenden Ansprüche, enthaltend mindestens ein kationisches Tensid in einem Gewichtsanteil von bis zu 5 Gew.-% am Gesamtgewicht des Haarbehandlungsmittels.

8. Haarbehandlungsmittel nach einem der vorhergehenden Ansprüche, enthaltend mindestens ein kationisches Polymer in einem Gewichtsanteil von 0,01 bis 3 Gew.-% am Gesamtgewicht des Haarbehandlungsmittels.

9. Haarbehandlungsmittel nach einem der vorhergehenden Ansprüche, enthaltend mindestens eine Öl-, Fett- oder Wachskomponente in einem Gewichtsanteil von 0,01 bis 5 Gew.-% am Gesamtgewicht des Haarbehandlungsmittels.

10. Haarbehandlungsmittel nach einem der vorhergehenden Ansprüche, enthaltend mindestens ein Proteinhydrolysat in einem Gewichtsanteil von 0,01 bis 3 Gew.-% am Gesamtgewicht des Haarbehandlungsmittels und/oder mindestens einen Pflanzenextrakt in einem Gewichtsanteil von 0,01 bis 3 Gew.-% am Gesamtgewicht des Haarbehandlungsmittels.

11. Verwendung einer Wirkstoffkombination umfassend 3-Hydroxypropyloctansäureester a) und mindestens einen von a) verschiedenen Antischuppenwirkstoff b),
wobei die Antischuppenwirkstoffe a) und b) in einem Gewichtsanteil von bis zu 0,5 Gew.-% am Gesamtgewicht des Haarbehandlungsmittels enthalten sind und
wobei der mindestens eine Antischuppenwirkstoff Piroctone Olamine enthält,
in Haarbehandlungsmitteln zur
a. Erzielung einer verbesserten Antischuppenwirksamkeit,
b. Optimierung der Viskositätseinstellung,
c. Stabilisierung der Schaumeigenschaften,
d. Verbesserung des Haargriffs,
e. Verbesserung der Elastizität der Haare,
f. Verbesserung der Geschmeidigkeit der Haare.

## Claims

1. Hair treatment agent containing
a) 3-hydroxypropyloctanoic acid ester (propanediol caprylate) and
b) at least one of a) various anti-dandruff agents
wherein the anti-dandruff agents a) and b) in a weight proportion of up toare contained and 0.75% by weight of the total weight of the hair treatment agent
wherein at least one anti-dandruff agent b) contains piroctone olamine.

2. Hair treatment agent according to claim 1, containing as anti-dandruff agent b) a mixture of piroctone olamine and at least one zinc salt selected from the group consisting of zinc pyrithione, zinc oxide, and/or zinc carbonate.

3. Hair treatment agent according to one of claims 1 or 2, containing 3-hydroxypropyloctanoic acid ester and the anti-dandruff agent b) in a weight ratio of 1:3 to 3:1.

4. Hair treatment agent according to one of the preceding claims, it contains at least one anionic, amphoteric, zwitterionic, nonionic, and/or cationic surfactant in a weight proportion of up to 15% by weight of the total weight of the hair treatment agent.

5. Hair treatment agent according to one of the preceding claims, containing at least one anionic surfactant in a weight proportion of up to 12.5% by weight of the total weight of the hair treatment agent.

6. Hair treatment agent according to one of the preceding claims, containing
(i) at least one anionic surfactant in a weight proportion of 1 to 10% by weight of the total weight of the hair treatment agent and
(ii) at least one amphoteric and/or zwitterionic surfactant in a weight proportion of 0.1 to 5% by weight of the total weight of the hair treatment agent and/or
(iii) at least one nonionic surfactant in a weight proportion of 0.1 to 5% by weight of the total weight of the hair treatment agent.

7. Hair treatment agent according to one of the preceding claims, containing at least one cationic surfactant in a weight proportion of up to 5% by weight of the total weight of the hair treatment agent.

8. Hair treatment agent according to one of the preceding claims, contain at least one cationic polymer in a weight proportion of 0.01 to 3% by weight of the total weight of the hair treatment agent.

9. Hair treatment agent according to one of the preceding claims, containing at least one oil, fat, or wax component in a weight proportion of 0.01 to 5% by weight of the total weight of the hair treatment agent.

10. Hair treatment agent according to one of the preceding claims, containing at least one protein hydrolysate in a weight proportion of 0.01 to 3% by weight of the total weight of the hair treatment agent and/or at least one plant extract in a weight proportion of 0.01 to 3% by weight of the total weight of the hair treatment agent.

11. Use of an active ingredient combination comprising 3-hydroxypropyloctanoic acid ester a) and at least one anti-dandruff active ingredient b) different from a) ,
wherein the anti-dandruff agents a) and b) in a weight proportion of up to 0.75% by weight are contained of the total weight of the hair treatment agent and wherein the at least one anti-dandruff agent contains piroctone olamine, in hair treatment agents for
a. achieving improved anti-dandruff efficacy,
b. optimization of viscosity adjustment,
c. stabilizing the foaming properties,
d. improving hair texture,
e. improving hair elasticity,
f. Improving hair smoothness.

## Revendications

1. Produit de traitement capillaire contenant
a) des esters d'acide 3-hydroxypropyloctanoïque (caprylate de propanediol) et
b) au moins un des composés suivants : a) différents agents antipelliculaires
les agents antipelliculaires a) et b) dans une proportion en poids allant jusqu'àétant contenus et 0,75 % du poids total du produit de traitement capillaire
l'au moins un agent antipelliculaire b) contenant de la piroctone olamine.

2. Produit de traitement capillaire selon la revendication 1, contenant comme agent antipelliculaire b) un mélange de piroctone olamine et d'au moins un sel de zinc choisi dans le groupe constitué par la pyrithione de zinc, l'oxyde de zinc et/ou le carbonate de zinc.

3. Produit de traitement capillaire selon l'une des revendications 1 ou 2, contenant un ester d'acide 3-hydroxypropyloctanoïque et l'agent antipelliculaire b) dans un rapport pondéral de 1:3 à 3:1.

4. Produit de traitement capillaire selon l'une des revendications précédentes, contenant au moins un tensioactif anionique, amphotère, zwitterionique, non ionique et/ou cationique dans une proportion en poids allant jusqu'à 15 % du poids total du produit de traitement capillaire.

5. Produit de traitement capillaire selon l'une des revendications précédentes, contenant au moins un tensioactif anionique dans une proportion en poids allant jusqu'à 12,5 % du poids total du produit de traitement capillaire.

6. Produit de traitement capillaire selon l'une des revendications précédentes, contenant
(i) au moins un tensioactif anionique dans une proportion pondérale de 1 à 10 % en poids par rapport au poids total du produit de traitement capillaire et
(ii) au moins un tensioactif amphotère et/ou zwitterionique dans une proportion pondérale de 0,1 à 5 % en poids par rapport au poids total du produit de traitement capillaire et/ou
(iii) au moins un tensioactif non ionique dans une proportion pondérale de 0,1 à 5 % en poids par rapport au poids total du produit de traitement capillaire.

7. Produit de traitement capillaire selon l'une des revendications précédentes, contenant au moins un tensioactif cationique dans une proportion pondérale allant jusqu'à 5 % en poids par rapport au poids total du produit de traitement capillaire.

8. Produit de traitement capillaire selon l'une des revendications précédentes, contenant au moins un polymère cationique dans une proportion pondérale de 0,01 à 3 % en poids par rapport au poids total du produit de traitement capillaire.

9. Produit de traitement capillaire selon l'une des revendications précédentes, contenant au moins un composant huileux, gras ou cireux dans une proportion pondérale de 0,01 à 5 % du poids total du produit de traitement capillaire.

10. Produit de traitement capillaire selon l'une des revendications précédentes, contenant au moins un hydrolysat de protéines dans une proportion pondérale de 0,01 à 3 % en poids par rapport au poids total du produit de traitement capillaire et/ou au moins un extrait végétal dans une proportion pondérale de 0,01 à 3 % en poids par rapport au poids total du produit de traitement capillaire.

11. Utilisation d'une combinaison d'agents actifs comprenant un ester d'acide 3-hydroxypropyloctanoïque a) et au moins un agent antipelliculaire b) différent de a) ,
les agents antipelliculaires a) et b) dans une proportion en poids allant jusqu'à 0,75 % en poids étant contenus par rapport au poids total du produit de traitement capillaire et le au moins un agent antipelliculaire contenant de la piroctone olamine, dans des produits de traitement capillaire pour
a. obtenir une efficacité antipelliculaire améliorée,
b. optimiser le réglage de la viscosité,
c. la stabilisation des propriétés moussantes,
d. l'amélioration du toucher des cheveux,
e. améliorer l'élasticité des cheveux,
f. Amélioration de la souplesse des cheveux.
